# EUROPEAN PATENT APPLICATION

(11) **EP 1 990 617 A1**
(43) Date of publication of application: **12.11.2008**
(21) Application number: 07107813.3
(22) Date of filing: 09.05.2007
(51) Int. Cl.: G01J 3/42, G01N 21/35, G01J 3/28, G01N 21/27

(54) **Determination of tannins in vinefication products**

(71) Applicant: FOSS Analytical A/S, 3400 Hilleroed (DK)
(72) Inventor: Skibsted Jensen, Jacob, 2730, Herlev (DK); Malmborg Werge, Hans Henrik, 2800, Kgs. Lyngby (DK)
(74) Representative: Hilton, Charles Paul

(57) **Abstract**

An analyzer (2) comprises a spectrometer (6) for detecting mid-infrared electromagnetic radiation after its interaction with a test sample of a vinefication product and generates, as an output, spectral data dependent on the detected radiation. The analyzer (2) further comprises a data processor (8) that has access to a calibration model, typically stored on a mass memory device (30), linking spectral data to tannin content of calibration samples and used in the determination of tannins in the test sample from the output of the spectrometer (6). The calibration model is established using mid-infrared spectral data from the calibration samples correlated with the tannin content of the same samples having been determined using protein precipitation.

## Description

The present invention relates to an analyzer for and a method of the determination of tannins in vinefication products, including wine fermentations and the final wine product, by means of spectrometric analysis.

Today price conscious consumers want better wines at less money so the demands for the technology behind wine production increase. Year after year the growers and producers have to make as high and as homogenous a quality as possible.

One of the important parameters for the quality of a red wine is its content of polyphenols. Among other things the polyphenols contribute to the sensoric qualities of a red wine, for instance the colour, the sense of "roundness" and the "body" in the mouth in addition to a general impact on flavour, partially through bitterness, mouth drying sensation and flavour stability. The types of polyphenols that are present in red wine in the greatest amounts are a group of polyphenols called tannins. The tannins account for up to about the half of the content of polyphenols of a red wine.

Tannins as a group are defined by their ability to precipitate with protein, and thus have a great impact on the consumers sensoric experience of the wine because the tannins are responsible for the astringency of the red wine. When consuming a red wine the tannins bind to the salivary proteins and then precipitate to leave a mouth drying sensation. This sensation is generally recognized as a feeling of puckeriness and dryness in the palate and can be unpleasant at high concentrations of tannin. If there is very little tannin in the wine it can seem more reminiscent of fruit juice with alcohol. Thus the tannins are very important for the "balance" of a given red wine (i.e. the tastiness of a wine, how palatable it is), the structure and sense of "backbone". Furthermore the tannins have a conserving effect on the red wine. Tannins play a big role in the development of wine after fermentation, and are a primary factor in long time storage of wines (both in cask and bottle).

With this in mind it is clear that it will be very valuable for winemakers to know as much as possible about the phenolic structure of their grapes, their fermenting wines and the finished wines. The winemaker will know a lot about a given harvest already before the harvest has begun, he will have the option of choosing how to handle the grapes to ensure that the wine reaches the quality he wishes, and with respect to tannins he will be able to either add further tannins if the grapes are lacking or he will be able to do less extraction of the skins if they are too tannic. In many countries it is allowed to add tannins during wine production. The ability to measure polyphenols and tannins will thus be very valuable to growers, winemakers, large scale manufacturers, oenologists and those responsible for the purchase of grapes for big producers.

The determination of the concentration of phenolic compounds, including tannins, in red wine fermentations by near infrared (NIR) spectroscopy has been disclosed in a paper entitled "Prediction of phenolic compounds in red wine fermentations by visible and near infrared spectroscopy" by Cozzolino et al which was published in Analytica Chimica Acta 513 (2004) 73-80. Here a monochromator was used to generate spectral data from electromagnetic energy in the wavelength regions between 400 nm (25,000 cm⁻¹) and 2500 nm (4,000 cm⁻¹), that is from visible to NIR regions, having been transmitted through a sample of a red wine fermentation. This data was analysed using calibration models developed from standard chemometric techniques employing spectral data of reference fermentation samples, the phenolic content of each of which was established using high performance liquid chromatography (HPLC) analysis. The calibration models developed were however specific to the grape variety and showed sensitivity to growing and/or fermentation conditions.

According to one aspect of the present invention there is provided an analyzer comprising a spectrometer, preferably a Fourier Transform spectrometer, configured to detect radiation in the mid-infrared region of the electromagnetic spectrum after having interacted with, such as transmission through or reflection from, a test sample of a liquid vinefication product and to generate spectral data, such as wavelength dependent intensity variations, therefrom. The analyser also comprises a data processor having access to a calibration model linking features of the generated spectral data to the presence of tannins in the sample, the calibration model being established from calibration samples the tannin content of which having been determined using a protein precipitation assay and standard chemometric techniques.

It has been discovered that the combination of mid-infrared spectral data with reference data established using tannin contents of reference samples determined using a protein precipitation assay provides an unexpectedly robust and accurate calibration model and hence reproducible analysis results.

According to a further aspect of the present invention there is provided a method of analyzing liquid vinefication samples to establish a tannin content. The method comprises generating spectral data in a spectrometer from mid-infrared radiation detected after its interaction with a test sample of the liquid vinefication product; in a data processor processing the generated spectral data by applying to the data a calibration model which links tannin content of a liquid vinefication sample to spectral data in the mid-infrared to generate an indication of the tannin content of the sample; and providing an output signal from the data processor dependent on the generated indication. The method further comprises a calibration step in which the calibration model is established from mid-infrared spectral data of reference liquid vinefication samples the tannin content of which having been determined using a protein precipitation assay.

Exemplary embodiments of the present invention will now be described with reference to the figures of the accompanying drawings, of which:
Fig. 1 shows a schematic representation of an analyzer according to the present invention;
Fig. 2 shows a flow chart of a method of determining tannin content according to the present invention and employed in the analyzer of Fig. 1; and
Fig.3 shows a representative mid-infrared spectrum obtained from a wine sample by means of the analyzer of Fig. 1 as well as variations therein caused by tannin changes.

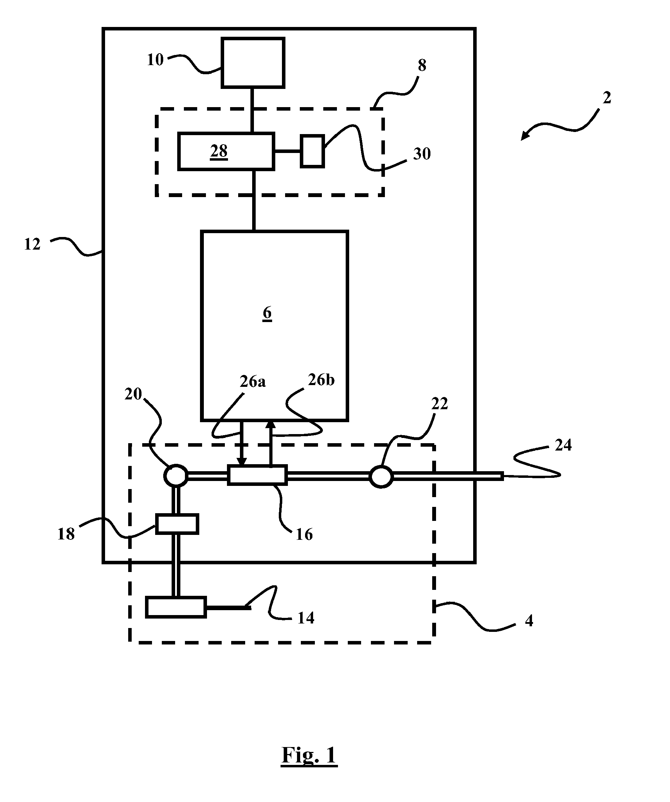

Considering now the analyzer 2 of Fig. 1, this analyzer 2 in the present example comprises a sample presentation means 4, a spectrometer 6, a data processor 8 and a display unit 10 all mainly contained within a housing 12.

The sample presentation means 4 here comprises an extraction needle 14 provided in flow communication with an optical sample cell 16 via a filter unit 18 and a first pump 20. The pump 20 operates in any known manner to automatically introduce a quantity, preferably a predetermined quantity, from a source (not shown) of liquid vinefication product, such as wine, into the optical sample cell 16 via the extraction needle 14.

A second pump 22 is, in the present example, also provided as part of the sample presentation means 4 of the present embodiment and operates to transport liquid vinefication product from the sample cell 16 and out of the analyzer via an outlet 24 after the necessary spectral information has been obtained by the spectrometer 6.

In the present embodiment the internal volume of the optical sample cell 16 is provided in optical communication with the spectrometer 6 using a delivery optical fibre 26a and a collection optical fibre 26b arrangement. In this manner broadband mid-infrared radiation from the spectrometer 6 is introduced to a liquid sample in the optical cell 16 via the fibre 26a and is collected after its interaction with the liquid sample to be provided to the spectrometer 6 via the fibre 26b.

The spectrometer 6 is configured to generate as an output spectral data, such as an electronic or digital representation of detected intensity variations as a function of wavelength, for the mid-infrared radiation provided to it. It will be appreciated that this spectrometer 6 may be of any known type such as a monochromator or a Fourier Transform spectrometer that is capable of generating such an output.

The output from the spectrometer 6 is provided as an input to a calculations unit 28 of the data processor 8. This calculations unit 28 is programmed using standard programming techniques to perform the appropriate calculations of a method of the present invention. A mass memory device 30, such as a hard disk or a CD ROM unit, is operably connected to the calculations unit 28. Data is provided to the calculations unit 28 from the mass memory device 30 to permit evaluation by the calculations unit 28 of the tannin content of a liquid vinefication product from the generated spectral data of a sample of that product in the optical sample cell 16 and in a manner according to the present invention. This data from the mass storage unit 30 may for example be in the form of a calibration model; coefficients for use in a calibration model already located in the calculations unit 28, or reference spectral data of calibration samples from which a calibration model can be constructed within the calculations unit 28, preferably in dependence of a comparison of the calibration spectral data with spectral data from the sample in the optical cell 16. Indeed the calibration model may be constructed in any known manner for use in the calculations unit 28.

The data processor 8 is configured to produce as an output an indication of the tannin content calculated in the calculations unit 28, for example as a numeric value indicating the level of tannin in the sample or as a quality index based on the so calculated tannin content. This output may be used in a number of different ways such as to drive a user interface such as the display unit 10 (as in the present embodiment) or a printer to provide a sensible indication of the tannin content, as a control signal in a fermentation process or to activate an alarm if the tannin content is beyond a predetermined threshold value. The output may also be stored, such as by the mass storage device 30, for later use or presentation. It will also be appreciated that in other embodiments according to the present invention some or all of the functionality of the data processor 8 may be provided in a separate, suitably configured and programmed personal computer.

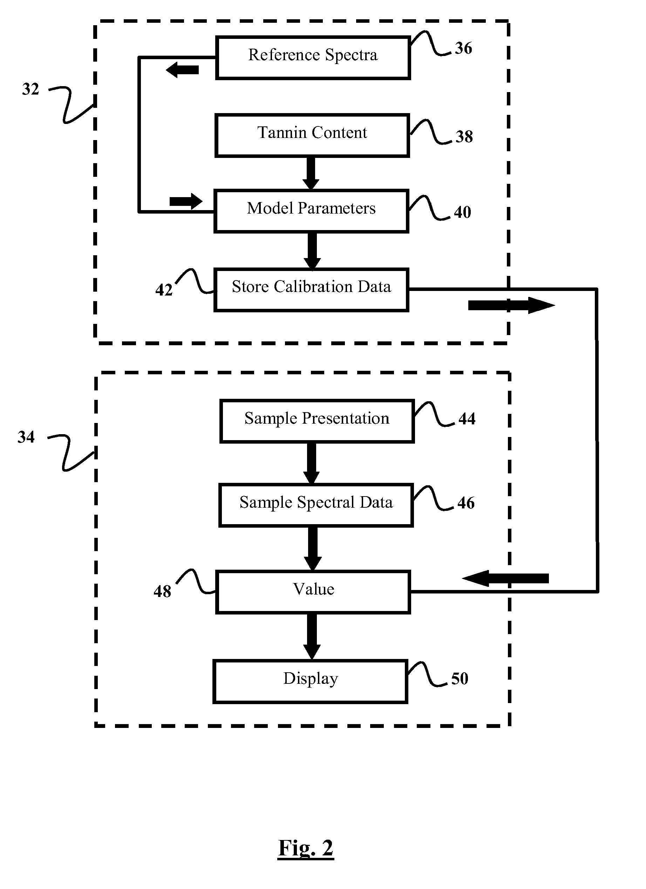

Turning now to Fig. 2 a flowchart is presented for an exemplary method for analysis according to the present invention and usable with the embodiment of the analyzer according to Fig. 1.

The method for analysis comprises a calibration step 32, during which data for a calibration model is generated from calibration samples and an analysis step 34, during which test samples are analysed using the so generated data to determine an indication of the tannin content of that test sample.

The calibration step 32 is typically but not necessarily performed at the site of manufacture of the analyzer 10 and is generally performed thereafter only occasionally, if at all. Of course it is possible for owners of the analyser 10 to perform this calibration step 32 (or one of more sub-steps thereof) in order to construct an individual calibration. This calibration step comprises in the present embodiment a number of sub-steps 36-42 during which reference spectra are collected from reference samples (sub-step 36); the tannin content of each of the reference samples is obtained using protein precipitation (sub-step 38); calibration model parameters are generated (sub-step 40) from the collected reference spectra and the correlated tannin contents obtained in the previous sub-steps 36, 38; and a storage sub-step 42 in which the parameters and/or the calibration model including parameters are committed to a mass storage 30 such as a hard disk or CD ROM. In some embodiments the generation step, sub-step 40, may be omitted and the reference spectra from sub-step 36 correlated with the tannin contents from sub-step 38 are stored (perhaps after being subjected to some form of data compression or other basic processing) directly on the mass storage 30. Generally information stored during this storage sub-step 42 is to be referred to as "calibration data".

Once the calibration step is completed and the data on the mass storage 30 is made accessible to the calculations unit 28 then the analyzer 2 is ready to perform analysis of liquid vinefication products during the analysis step 34.

This analysis step 34 in the present embodiment comprises a number of sub-steps 44-50 during which a test sample is presented to a spectrometer (sub-step 44) for analysis; the so presented sample is analysed to generate spectral data in the mid-infrared region of the electromagnetic spectrum (sub-step 46); a value of the tannin content of the sample is calculated (sub-step 48) in a data processor 8 having access to the generated spectral data and the stored calibration data; and an indication of the value is output at sub-step 50.

Considering again the calibration step 32, a non-limiting example will now be described in greater detail. Calibration samples for the calibration development were sourced from commercially available wines. The number and the characteristics of the different reference samples were selected to best represent the likely variation of test samples expected during the day-to-day operation of the analyzer 2 and in the present example were selected to provide a diverse sample set with respect to country of origin, grape variety and wine age. In the present example at least ten examples per class property were employed.

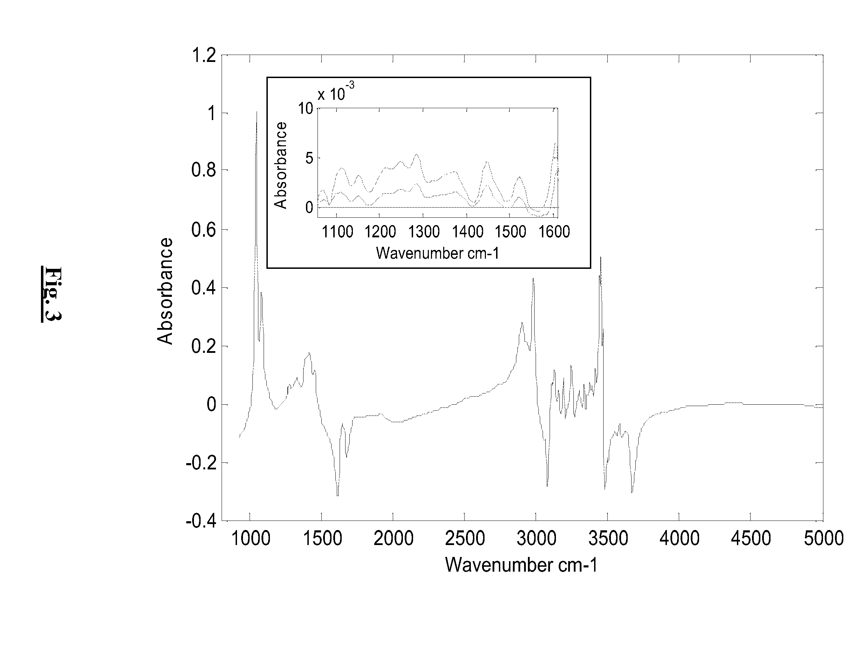

During the sub-step 36 mid-infra red transmission spectra were obtained for each of the calibration samples in the wavelength region between 5008 cm⁻¹ and 925 cm⁻¹ and as is illustrated in the representative spectrum of Fig. 3. As can be seen from this Fig. 3, tannin shows characteristic spectral activity primarily in the wavelength region between 1100 cm⁻¹ and 1600cm⁻¹ (shown by the insert of Fig. 3). As can be seen from the insert, as the tannin content increases so does absorbance in that spectral region. Particularly the insert to Fig 3 illustrates three absorbance spectral measurements from a wine sample spiked with 0, 1 and 2 g/L of commercially available tannin extract respectively (increasing absorbance) and clearly demonstrates that absorbance in that spectral region increases with tannin content.

During the sub-step 38 the tannin content of each of the calibration wines is determined using protein precipitation.

Hagerman and Butler suggested in 1978 a method that involved the precipitation of the tannins with the protein bovine serum albumin (BSA). When adding this protein to a red wine or a red wine solution it was suggested that the only precipitate would be the tannin-protein complex. This precipitate was isolated and redissolved with Sodium Dodecyl Sulphate (SDS) in an alkaline solution. The tannin content was determined by adding a solution of Ferric Chloride to the SDS-solution and the absorption at 510 nm was measured. The measurement was correlated to the absorption of (+)-Catechin (CAE) and thus the tannin content of a red wine was measured in Catechin equivalents (mg CAE/L).

In 2003 James F. Harbertson suggested that the BSA based assay could be enhanced to also measure the content of polymeric pigments (PPs) of a red wine. This had previously been done on its own via bleaching with bisulphite. In short Harbetson measured the PPs by first measuring the total colour of the wine (the total colour from both anthocyanins and PPs) and then bleaching it with bisulphite. This removed the colour contribution from the anthocyanins and left only the colour contributed to the wine from PPs. When tannin was precipitated from the wine with the addition of BSA, the large polymeric pigments (LPPs) were precipitated as well. However the small polymeric pigments (SPPs) were still dissolved in the solution along with the anthocyanins. This solution was bleached with bisulphite so that the only remaining colour was that of the SPPs. In this way it was possible to measure the content of SPPs and to calculate the content of LPPs.

Based on the above experimental reports a BSA assay was established that, in the present exemplary embodiment, comprised the following steps:
1) An Eppendorf tube of each of the 128 wines was obtained.
2) From each Eppendorf tube 0.8 mL wine was mixed with 0.8 mL pH 3.3 Buffer in a new Eppendorf tube. This Eppendorf tube was shaken to achieve a homogenous mix of wine and buffer. For wines where an initial screening showed high tannin content only 0.5 mL of wine was mixed with 1 mL or 1.5 mL of the pH 3.3 Buffer dependant on how high the tannin content was. For the wines that were low in tannin content either 1 mL or 1.5 mL wine was mixed with 0.5 mL pH 3.3 Buffer.
3) 0.5 mL of 2) was pipetted off and mixed with 1 mL of a 1 mg/mL BSA solution in a new Eppendorf tube and this was inverted for 30 minutes.
4) While 3) is inverting for 30 minutes another 0.5 mL of 2) was pipetted off and mixed with pH 4.9 Buffer in a new Eppendorf tube and 1 mL of this solution was transferred with a pipette to a spectrophotometer cuvette.
5) After 10 minutes the absorption of the solution in 4) was measured at 520 nm. This is the measurement of the Total Pigments content.
6) The cuvettes in 5) were then added 80 µL of a Harbertson Bisulphite solution and the absorptions were measured after 10 minutes at 520 nm. This is the measurement of the Polymeric Pigments (PPs) content. Steps 4), 5) and 6) altogether lasts about 30 minutes.
7) The Eppendorf tube from 3) that has now been inverted for 30 minutes is centrifuged for 5 minutes at 14000 g.
8) 1 mL of the supernatant is transferred to a new spectrophotometer cuvette and put aside for the time being. The rest of the supernatant is discarded.
9) The precipitate (or 'pellet') is washed with 0.2 mL of pH 4.9 Buffer. This is then centrifuged for 3 minutes and the supernatant is discarded. This wash is repeated once.
10) The pellet is mixed with 1.5 mL of an SDS/TEA solution and the tube is inverted for 20 minutes.
11) While 10) is inverting the cuvettes from 8) are added 80 µL of the Harbertson Bisulphite solution. After 10 minutes the absorption was measured at 520 nm. This measurement represents the content of Small Polymeric Pigments (SPPs). This measurement also allows for calculation of Large Polymeric Pigments (LPPs). This is a product of the difference between PP content and the SPPs content.
12) The Eppendorf tubes from 10) are removed from the inverter after the 20 minutes. 1 mL of each of the solutions is transferred to a spectrophotometer cuvette and the absorption was measured at 510 nm. This is the Background measurement.
13) The cuvettes were then added 125 µL of the FeCl₃ solution and after 10 minutes the absorptions were measured at 510 nm. The tannin response was calculated as 1.125 times the final absorbance minus the background absorbance. The Tannin content was expressed as mg catechins equivalents (CAE) per L from a standard curve of the color reaction between catechin and ferric chloride.

It will be appreciated that the order in which the mid-infrared spectra are obtained (sub-step 36) and the tannin content measured (sub-step 38) can be reversed or carried out simultaneously without diverging from the invention as claimed.

At sub-step 40 a Partial Least Squares (PLS) calibration was established using the results of the measurements made on the calibration sample set at sub-steps 36 and 38. The calibration employed ten PLS factors (as for example described by Ebsen, Kim H., Multivariate Data Analysis - in practice 4th Edition, Camo Inc., PO Box 1628, Oregon, USA (2000)) and it is cross validated using ten segments. This means that the prediction is always employed on 1/10 of the set. The IR spectrum of each wine is assigned with the numeric tannin amount of the wine in mg CAE /L. The best range of the mid-infrared spectrum for the tannin prediction is here chosen through trial and error. It is found that the best prediction is achieved in the in the wavelength region between 1156.5 cm⁻¹ and 1576.7 cm⁻¹.

It will be appreciated that known multivariate analysis techniques other than PLS may be employed to establish a calibration. Here multivariate methods refer to evaluation methods utilizing more than just one measurement signal of a same sample in order to arrive at an analysis result and include Multi-linear Regression (MLR), Principal Components Analysis (PCA), clustering methods, and artificial neural networks.

Typically in such multivariate analysis techniques about 70% of the totality of the calibration wine set measured per class are used to establish calibration data representing the calibration model and about 30% are used to validate the so established calibration model. In the present exemplary embodiment and by way of example only, eighty one members of the wine set were used to establish the calibration and forty seven members were used for validation.

The following solutions were employed in the exemplary BSA assay described above:
pH 3.3 Buffer: This was made of 10.0 grams of tartaric acid, that was transferred to a 2 L glass beaker using de-ionized water. 250 mL ethanol was added and the solution was diluted to approximately 1.6 L. pH was adjusted to 3.3 using both 1 M NaOH_{(aq)} and 4 M NaOH_{(aq)}. This solution was transferred quantitatively to a 2000 mL volumetric flask and diluted to 2000 mL.
pH 4.9 Buffer: This was prepared from 9.93 g NaCl₍ₛ₎ transferred to a 1 L glass beaker with de-ionized water. 12.01 g Glacial acetic acid was weighed off and added to the solution. The volume was increased to approximately 0.8 L and pH was adjusted to 4.9 using 1 M NaOH(aq). The solution was transferred quantitatively to a 1000 mL volumetric flask and volume was increased to 1000 mL.
Harbertson Bisulphite solution: 16.0 g K₂S₂O₅₍ₛ₎ was weighed off and transferred quantitatively to a 200 mL volumetric flask. It was dissolved and volume was increased to 200 mL using de-ionized water to give at total of 8 % (w/v) of potassium bisulphite.
SDS/TEA solution: This was prepared from 11.2 g Triethanol Amine₍ₗ₎ and 10.0 g Sodium Lauryl Sulphate₍ₛ₎. Both were transferred to glass beaker using de-ionized water and volume was increased to approximately 0.16 L and then the solution was stirred for a couple of hours as the SDS took a long time to dissolve. The pH was adjusted to 9.4 using 1 M HCl_{(aq)}. The solution was transferred quantitatively to a 200 mL volumetric flask and volume was increased to 200 mL to give a final concentration of 5% (v/v) of TEA and 5% (w/v) of SDS.
FeCl₃ solution: An 11.4 mM FeCl₃·6H₂O₍ₛ₎ solution was prepared from 308.9 mg FeCl₃·6H₂O₍ₛ₎ and 1.143 mL 1 M HCl_{(aq)} and diluted in a volumetric flask to a volume of 100 mL.
1.0 mg/mL BSA solution: 0.100 g Bovine Serum Albumin was weighed off and transferred quantitatively to a 100 mL volumetric flask and volume was increased to 100 mL using the pH 4.9 Buffer.
2.0 mg/mL BSA solution: 0.2000 g Bovine Serum Albumin was weighed off and transferred quantitatively to a 100 mL volumetric flask and volume was increased to 100 mL using the pH 4.9 Buffer.

It will be appreciated by those skilled in the art that the assay, particularly the compositions of the various solutions, may be varied without departing from the invention as claimed.

## Claims

1. An analyzer (2) comprising a spectrometer (6) for detecting electromagnetic radiation after its interaction with a test sample of a vinefication product and generating as an output spectral data dependent on the detected radiation; and a data processor (8) having access to a calibration model linking spectral data to tannin content of calibration samples of vinefication product and being configured to process the output spectral data using the calibration model to generate one or both a qualitative and a quantitative indication of tannins in the test sample and to provide an output signal dependent on the generated indication **characterised in that** the spectrometer (6) is configured to detect radiation in the mid-infrared region of the electromagnetic spectrum **and in that** the calibration model is established using mid-infrared spectral data from the calibration samples correlated with the tannin content of the same samples having been determined using protein precipitation.

2. A method for the analysis of a tannin content of a liquid vinefication product by spectrophotometric analysis comprising a calibration step (32) during which calibration data is established from a calibration sample set of liquid vinefication products; and an analysis step (34) in which the calibration data is applied in a data processor to the analysis of spectral data obtained by a spectrometer from a test sample of a liquid vinefication product and to provide as an output an indication of the tannin content of the test sample
**characterised in that** the calibration step comprises obtaining a reference measure of tannin in each calibration sample of the calibration sample set by means of protein precipitation assay (38) and obtaining associated mid-infrared spectral information for each of the calibration sample set (36) **and in that** the analysis step (34) comprises generating in the data processor spectral intensity information from the mid-infrared region of the electromagnetic spectrum (46) as the spectral data.

3. A method as claimed in claim 2 **characterised in that** the protein precipitation assay (38) is a bovine serum albumin (BSA) assay.
